Europäisches Patentamt

European Patent Office

Office européen des brevets

(19)

(11) EP 1 253 856 B1

(12) **EUROPEAN PATENT SPECIFICATION**

(45) Date of publication and mention
of the grant of the patent:
**30.03.2005 Bulletin 2005/13**

(51) Int Cl.⁷: **A61B 8/08**, A61B 8/06,
A61B 5/00

(21) Application number: **01904142.5**

(22) Date of filing: **09.02.2001**

(86) International application number:
**PCT/GB2001/000549**

(87) International publication number:
**WO 2001/058358 (16.08.2001 Gazette 2001/33)**

(54) **APPARATUS FOR IN VIVO MONITORING OF THE EFFECT OF ANTIANGIOGENIC DRUGS ON CANCERS**

GERÄT ZUR IN-VIVO ÜBERWACHUNG DER WIRKUNG ANTIANGIOGENER ARZNEIMITTEL BEI KREBS

APPAREIL DE SURVEILLANCE IN VIVO DE L'EFFET DE MEDICAMENTS ANTI-ANGIOGENIQUES SUR DES CANCERS

(84) Designated Contracting States:
**AT BE CH CY DE DK ES FI FR GB GR IE IT LI LU MC NL PT SE TR**

(30) Priority: **11.02.2000 GB 0003032**

(43) Date of publication of application:
**06.11.2002 Bulletin 2002/45**

(73) Proprietor: **Highland Innovation Centre Limited Inverness, IV3 8NE (GB)**

(72) Inventor: **Watmough, David J.**
**Inverness IV3 8NE (GB)**

(74) Representative: **Jenkins, Peter David et al**
**PAGE WHITE & FARRER**
**54 Doughty Street**
**London WC1N 2LS (GB)**

(56) References cited:
**US-A- 5 007 428**

• ZHU Q: "IMAGER THAT COMBINES NEAR-INFRARED DIFFUSIVE LIGHT AND ULTRASOUND" OPTICS LETTERS,OPTICAL SOCIETY OF AMERICA, WASHINGTON,US, vol. 24, no. 15, 1 August 1999 (1999-08-01), pages 1050-1052, XP000859237 ISSN: 0146-9592

• BRITTENDEN J ET AL: "Preliminary clinical evaluation of a combined optical Doppler ultrasound instrument for the detection of breast cancer" BRITISH JOURNAL OF RADIOLOGY, DEC. 1995, BRITISH INST. RADIOL, UK, vol. 68, no. 816, pages 1344-1348, XP001005950 ISSN: 0007-1285 cited in the application

• WATMOUGH ET AL: "Study of tissue compressionin breast phantome" EUROPEAN JOURNAL OF SURGICAL ONCOLOGY, vol. 18, 1992, pages 538-544, ACADEMIC PRESS LTD

• WATMOUGH ET AL: "Does breast cancer screening depend on a wobbly hypothesis?" JOURNAL OF PUBLIC HEALTH MEDICINE, vol. 19, no. 4, 1997, pages 375-379,

• WATMOUGH: "Medical Imaging" 1983, SURREY UNIVERSITY PRESS Chapter 6 Diaphanography * pages 217-225 *

• WATMOUGH: "Diaphanography; Mechanism responsible for the images" ACTA RADIOLOGICA ONCOLOGY, vol. 21, 1982, pages 11-15,

• WELLS ET AL: "Tumour detection by ultrasonic Doppler blood-flow signals" ULTRASONICS, vol. 15, no. 2, 1 September 1977 (1977-09-01), pages 231-232, IPC SCIENCE AND TECHNOLOGY PRESS LTD, GUILDFORD, GB

EP 1 253 856 B1

**Description**

The present invention relates to an apparatus for examining a body of living tissue.

The use of an instrument combining light and Doppler Ultrasound to detect breast cancer [US Patent 5,007,428 Watmough] has been previously described. A preliminary study using the device (Brittenden J. Watmough D.J. Heys S.D. and Eremin O. [1995] Preliminary clinical evaluation of a combined optical Doppler ultrasound instrument for the detection of breast cancer. Brit. J. Radiol. 68, 1344 - 1348) on a series of patients with a variety of breast conditions demonstrated that 73% of cancers could be detected. It was not initially appreciated that the camera, tumour and light source should be placed on a common axis to achieve optimum sensitivity. Therefore it is expected that in future studies where this requirement is achieved from the outset that higher detection rates will be achievable. The optical examination reveals the presence of the cancer by virtue of excess absorption due to the presence of angiogenesis [i. e. blood vessels induced by the cancer to supply it with additional oxygen and nutrients for more rapid growth] around the periphery of the tumour. However haematoma and bruising can give rise to images which can be mistaken for cancers. The ultrasound Doppler examination was incorporated to exclude these latter conditions by virtue of the fact that no blood flow is associated with them. In such cases no Doppler frequency shift signals can be elicited from the periphery of the lesion. Most of the cancers which went undetected by the optical and Doppler ultrasound based instrument were small and had not developed neovascularisation or were demonstrated on X-ray solely by the presence of microcalcification. The important point to note here is that the optical / Doppler instrument worked well on virtually all larger advanced cancers.

Recently the idea of treating advanced cancers with angiogenesis inhibiting drugs has been proposed with a view to starving the cancer of oxygen and nutrients and therefore preventing further growth and hopefully shrinking it. The proposed new method of treatment has another potential advantage namely that any secondary tumours should be treated simultaneously.

Brittenden J *et al*: 'Preliminary clinical evaluation of a combined optical Doppler ultrasound instrument for the detection of breast cancer' British Journal of Radiology, Dec. 1995, British Inst. Radiol, UK, vol. 68, no. 816, pages 1344-1348, XP001005950 ISSN: 007-1285 discloses an apparatus for examining a body of living tissue having the features of the pre-characterising portion of claim 1.

US-A-5,007,428 discloses a combined Optical/Doppler apparatus for detection of cancer comprising an illuminator for application of light to tissues and, separately and independently, a probe for interrogation of a tumour by ultrasound.

Zhu Q: 'Imager that combines near-infrared diffusive light and ultrasound' Optics Letters, Optical Society of America, Washington, US, vol. 24, no. 15, 1 August 1999 (1999-08-01), pages 1050-1052, XP000859237 ISSN: 0146-9252 discloses the use of ultrasound and light to image a cancer of the breast.

There is a requirement for an instrument to monitor the cancer at intervals over a protracted period and to reveal the extent of neovascularisation and changes in it.

The present invention accordingly provides an apparatus for examining a body of living tissue containing a cancerous tumour the apparatus comprising an optical applicator having a light source for illuminating the tissues with electromagnetic radiation, a radiation receiving means for receiving the radiation transmitted through the tissues, means for producing an image of the received radiation, an ultrasound applying means (1,2) for applying ultrasound to a region surrounding the tumour, an ultrasound receiving means (3,4) for receiving reflected ultrasound from an advancing front of the cancerous tumour, means for producing a signal which is representative of the velocity of blood flow around the tumour and means for generating from the signal a graphical representation which provides an indication of angiogenesis and associated blood flow, characterised in that the ultrasound applying means (1,2) is incorporated in the optical applicator.

Preferably, the ultrasound applying means and the ultrasound receiving means comprise an ultrasonic Doppler bloodflow detector including piezoelectric transmitting and receiving elements which are incorporated in a housing of a light guide of the optical applicator.

Preferably, the apparatus further comprises a computer and a switching device, the switching device connecting the radiation receiving means and the ultrasound receiving means to the computer the switching device being selectively switchable between the radiation receiving means and the ultrasound receiving means thereby to select which of the radiation receiving means and the ultrasound receiving means is connected to the computer.

Preferably, the ultrasound applying means and the ultrasound receiving means (3,4) have n piezoelectric elements (1,2,3,4) where n = 2,4,6,8,10,12, ... and said piezoelectric elements are operated in pairs so as to obtain ultrasound Doppler signals from a hemispherical tissue volume around the tumour being monitored.

Preferably, the piezoelectric elements are operated in diagonally opposite pairs or the piezoelectric elements are operated in adjacent pairs.

Preferably, the ultrasound applying means and the ultrasound receiving means have piezoelectric elements and the geometry of the piezoelectric elements is in the form of annular segments or a series of circular discs all of which may in a plane at right angles to an optical axis of the optical applicator or inclined to that plane at angles up to about 45 degrees.

The piezoelectric elements may be operated in alternate pairs, and alternate pairs operate at 8 and 10 MHz or 10 MHz and 12 MHz or other similar frequency combinations.

Preferably, the apparatus further comprises an incrementing means for incrementing in steps the electromagnetic optical radiation from the light source.

Preferably, the apparatus further comprises means for comparing Doppler frequency shift spectra from serial examinations.

FIG. 1 shows an image obtained by transillumination recorded by a high sensitivity [moonlight] video camera;

FIG. 2 is an isometric display of Doppler frequency shift signals from the angiogenesis around an advanced cancer;

FIG. 3(a) is a sectional drawing of a light guide surrounded by Doppler ultrasound transducers 1, 2, 3 and 4;

FIG. 3(b) shows how a biggger fraction of tumour surface can be interrogated by changing the shape of the Doppler transducers;

FIG. 4 shows the arrangement in vertical section;

FIG. 5 shows by superposition how the light guide and the transducers are arranged with respect to the cancer.

A modified version of the optical/ultrasound Doppler instrument described in US Patent 5,007,428 is ideal for use in the apparatus of the present invention.

The present patent application describes an instrument capable of producing images of cancerous tumours and Doppler ultrasound frequency shift spectra, deriving from blood flow around them, which would be identical on successive occasions unless the antiangiogenesis drugs were having an effect on the extent of neovascularisation.

In the earlier study it was found that the angiogenesis around a cancer is not homogeneous and that using a small conventional Doppler probe with two typically millimetre-sized elements fails to produce Doppler shift spectra which can be meaningfully compared. It is necessary to devise a means of replacing the Doppler transducers [transmitter and receiver] in the same position in relation to the cancer on successive occasions. The direction of the transducer axes must also be reproduced on each occasion. The present application describes an instrument by means of which serial examinations of cancers using light and Doppler ultrasound can reveal the efficacy of treatments. Serial examinations by light show the image of the cancer diminishing in area over time as a result of successful therapy. The Doppler frequency shift spectra show diminishing signal amplitude and reduced values of frequency shift maxima, corresponding to the shrinking and or disappearance of vessels around the tumour. Complete destruction of neovascularisation leads to disappearance of the associated ultrasound Doppler frequency shift signals.

On previous occasions when using a conventional Doppler probe to interrogate a cancer serially [e. g. weekly intervals], the signals could not be interpreted in terms of changes in angiogenesis because there was no means of being sure that the position of the probe was the same on each occasion.

The transcutaneous measurement of blood flow velocity by ultrasound Doppler frequency shift is a well established technique, Wells *et al*, "Tumour detection by ultrasonic Doppler blood flow signals", Ultrasonics, 15 [1977], 231.

When an ultrasonic wave of frequency f and velocity c is reflected by blood flowing with velocity v, the Doppler shift $f_D$ is given by:

$$f_D = f_r - f$$

where $f_r$ is the received frequency.

It can be shown that

$$f_D = - (2 f \cdot v \cdot \cos x) / c$$

where x is the angle between the direction of flow and the axis of the ultrasound beam, and c the velocity of ultrasound is much greater than v.

In soft tissues and in blood $c = 1500$ m s$^{-1}$, and for

$$f = 8 \text{ MHz then } f_D = 2 \text{ kHz when } v = 100 \text{ mm s}^{-1}$$

and cos x = 1.

Transducers a few mm in diameter can be used to explore flow through a single vessel in a selective and sensitive fashion. In our study we examine signals generated by a chaotic mass of vessels (rather than a single one) and this is the reason for a spectrum of frequency shifts. Cancers give rise to high amplitude signals and high flow velocities (notably from arteriovenous shunts). Another characteristic of flow around tumours is positive (non-zero) flow throughout the cardiac cycle.

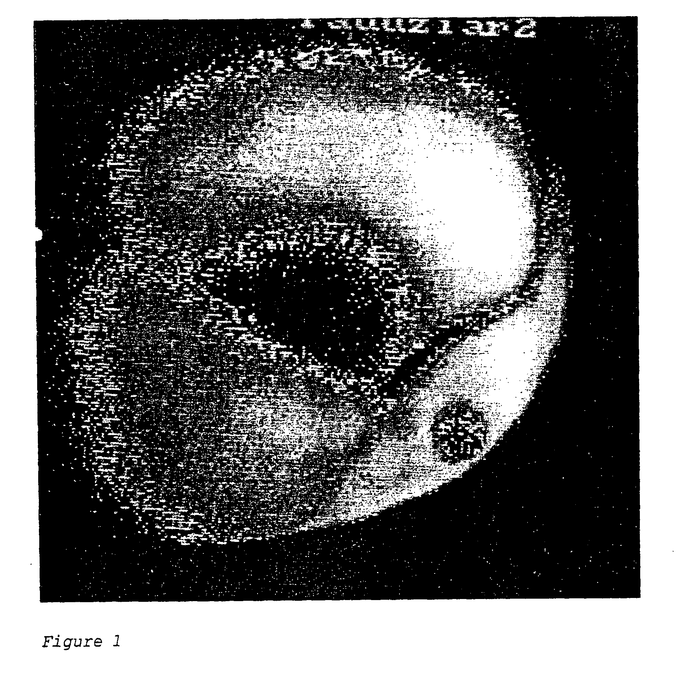

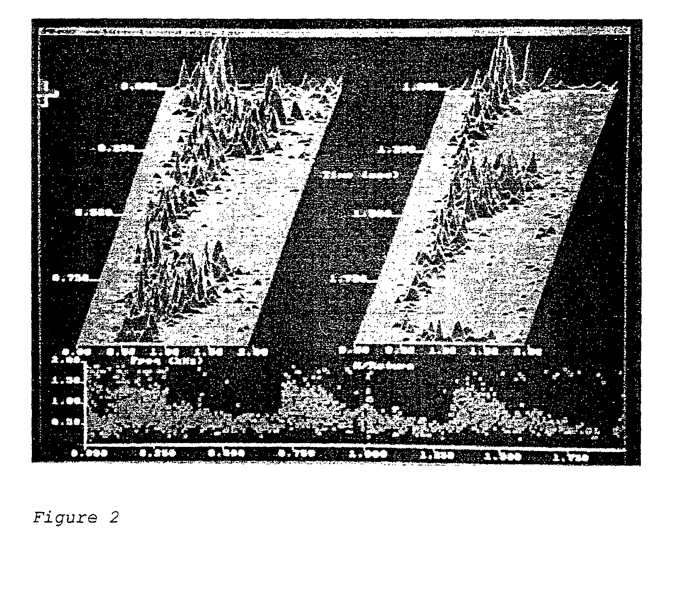

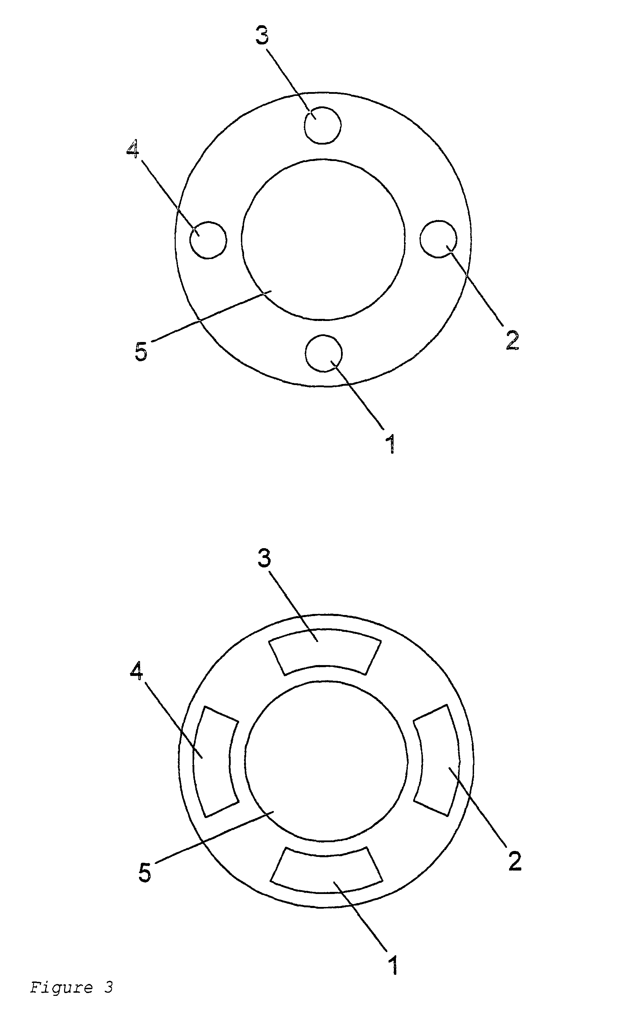

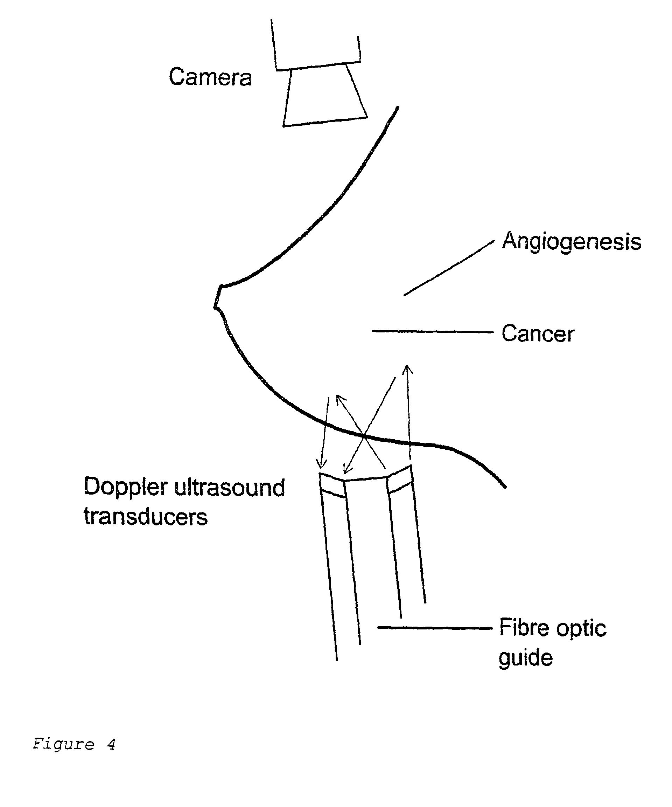

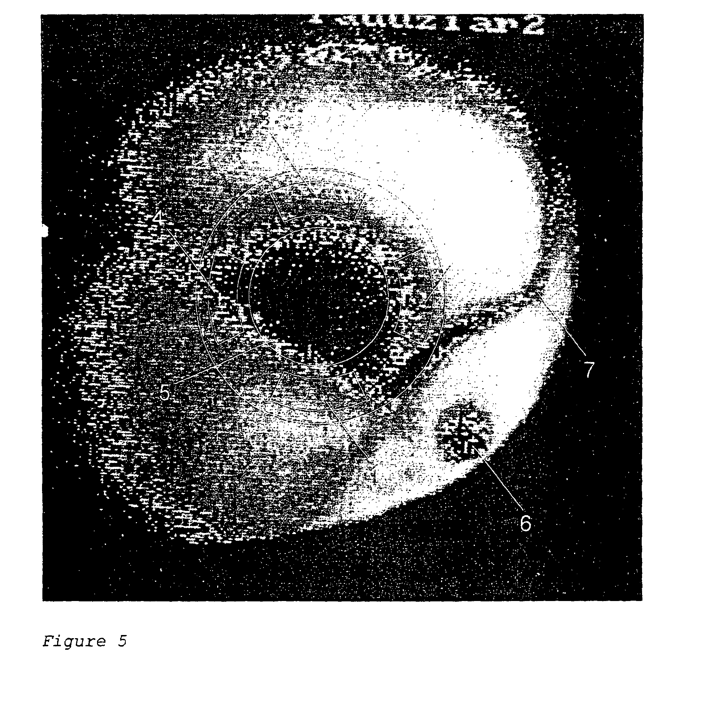

The invention is described by way of example. Figure 1 shows an image obtained by transillumination recorded by a high sensitivity [moonlight] video camera as described in US Patent 5,007,428 Watmough. The large dark area centrally situated is due to light absorption by angiogenesis surrounding a cancer about 2 cm deep in breast tissue. The nipple [small round dark area] and the superficial blood vessel [black line] on the right of the picture are typical features of optically produced images. Figure 2 is an isometric display of Doppler frequency shift signals from the angiogenesis around an advanced cancer. The characteristic features of malignancy described above are present. Figure 3(a) is a sectional drawing of a light guide [5] surrounded by Doppler ultrasound transducers 1, 2, 3 and 4. The combined optical Doppler ultrasound probe is normally placed on the underside of the breast and the light intensity, arranged to be incremented in steps, is varied until an image such as that in Figure 1 is obtained. The circular disc shaped piezoelectric Doppler transducers are activated so that [1] and [2] transmit and [3] and [4] receive. The difference frequency $f_D$ measures the blood flow in a large fraction of the advancing front of the tumour so that inhomogeneities in neovascularisation are unimportant. Figure 3 (b) shows how a bigger fraction of tumour surface can be interrogated by changing the shape of the Doppler transducers. The physical combination of the optical and ultrasound Doppler techniques in a single probe enable serial examinations to be carried out on a patient with the transducers replaced at the same site on each occasion. Where this condition is not met the ultrasound frequency shift spectra will not in general be comparable and no conclusions about the effect of antiangiogenic drugs can be drawn. Figure 4 shows the arrangement in vertical section. Note the angulation of the Doppler transducers towards the tumour and the arrows indicating reflection of ultrasound waves from the chaotic mass of vessels onto the receiving transducer (s). Although four transducers have been described any even number can be used. The inputs and outputs can be electronically processed in pairs or in parallel arrays. The preferred operating frequencies are 6, 8, 10 and 12 MHz and there may be several different probes associated with each instrument or in a single probe there can be opposite pairs of piezoelectric elements chosen to operate at different frequencies i.e. one pair at 8 MHz and another at 12 MHz. The reason for the choice of different frequencies is that the tumour depth may vary from patient to patient and 8MHz may be optimum in one case and 12 MHz in another. Whatever frequency is chosen must be maintained for each of the serial examinations unless two frequencies are chosen for each examination. Figure 5 shows by superposition how the light guide and the transducers are arranged with respect to the cancer. Only when the light is directed from the same position and at the same intensity will comparable images be obtained. The light intensity must therefore be measured and the numerical value must be stored and shown on the same monitor as the image. New examinations will start by displaying the previous image and varying the probe position until the geometry of a new image resembles as closely as possible the former one. A display where the former and present image are seen side by side is preferred. Landmarks such as nipple and supercial vessels not associated with angiogenesis may be used to locate the probe. In both images the intensity of illumination must have been maintained constant. Differences, such as the size of the dark area in Figure [1], will then correspond to the effect of drugs on the angiogenesis.

Disappearance of blood vessels comprising the angiogenesis will cause the dark area to diminish or vanish altogether. The above procedures will ensure that the Doppler ultrasound examination produces changes in spectra which can be interpreted in terms of the effect of the drugs. Details of signal processing using a computer or dedicated microprocessor are known in the established art. See for instance US Patent 5,007,428 Watmough and Wells PNT [1978] Biomedical Ultrasonics, Academic Press. What has not been taught by the prior art is how to monitor a cancer [breast or testicular] by combined optical and Doppler ultrasound means in such a way that the tests reveal the effect of antiangiogenic and other chemotherapeutic drugs over a protracted period or indefinitely.

References

[1] Watmough D.J. [1983]. Diaphanography. Chapter 6 in the book Medical Imaging [Editor Daphne Jackson ] Surrey University Press, 217-225.
[2] Watmough D.J. [1982]. Diaphanography; Mechanism responsible for the images. Acta. Radiologica Oncol. 21, 11-15.
[4] Watmough D.J., Quan K.M., Aspden R.M., and Mallard J.R. [1992] Phantom study of tissue compression: possible implications for the use of X- ray mammography as a method of imaging breast carcinoma. Europ. J. Surg. Oncol. 18, 538 - 544.
[5] Watmough DJ, Bhargava S, Memon A, Roy S, Syed F [1997] Does breast cancer screening depend on a wobbly hypothesis? Journal Public Health Medicine. 19 No. 4, 375-379.

**Claims**

1. An apparatus for examining a body of living tissue containing a cancerous tumour the apparatus comprising an optical applicator having a light source for illuminating the tissues with electromagnetic radiation, a radiation receiving means for receiving the radiation transmitted through the tissues, means for producing an image of the received radiation, an ultrasound applying means (1,2) for applying ultrasound to a region surrounding the tumour, an ultrasound receiving means (3,4) for receiving reflected ultrasound from an advancing front of the cancerous tumour, means for producing a signal which is representative of the velocity of blood flow around the tumour and means for generating from the signal a graphical representation which provides an indication of angiogenesis and associated blood flow, **characterised in that** the ultrasound applying means (1,2) is incorporated in the optical applicator.

**2.** An apparatus according to claim 1 wherein the ultrasound applying means (1,2) and the ultrasound receiving means (3,4) comprise an ultrasonic Doppler bloodflow detector including piezoelectric transmitting and receiving elements (1,2,3,4) which are incorporated in a housing of a light guide (5) of the optical applicator.

**3.** An apparatus according to claim 1 or claim 2 further comprising a computer and a switching device, the switching device connecting the radiation receiving means and the ultrasound receiving means (3,4) to the computer the switching device being selectively switchable between the radiation receiving means (3,4) and the ultrasound receiving means (3,4) thereby to select which of the radiation receiving means and the ultrasound receiving means is connected to the computer.

**4.** An apparatus according to any one of claim 1, claim 2 or claim 3 wherein the ultrasound applying means (1,2) and the ultrasound receiving means (3,4) have n piezoelectric elements (1,2,3,4) where n = 2,4,6,8,10,12, ... and said piezoelectric elements are operated in pairs so as to obtain ultrasound Doppler signals from a hemispherical tissue volume around the tumour being monitored.

**5.** An apparatus according to claim 4 wherein the piezoelectric elements (1,2,3,4) are operated in diagonally opposite pairs.

**6.** An apparatus according to claim 4 wherein the piezoelectric elements (1,2,3,4) are operated in adjacent pairs.

**7.** An apparatus according to any foregoing claim, wherein the ultrasound applying means (1,2) and the ultrasound receiving means (3,4) have piezoelectric elements (1,2,3,4) and the geometry of the piezoelectric elements (1,2,3,4) is in the form of annular segments or a series of circular discs all of which may in a plane at right angles to an optical axis of the optical applicator or inclined to that plane at angles up to about 45 degrees.

**8.** An apparatus according to claim 4, or any one of claims 5 to 7 when appendant on claim 4, wherein the piezoelectric elements (1,2,3,4) are operated in alternate pairs, and alternate pairs operate at 8 and 10 MHz or 10 MHz and 12 MHz or other similar frequency combinations.

**9.** An apparatus according to any forgoing claim further comprising an incrementing means for incrementing in steps the electromagnetic optical radiation from the light source.

**10.** An apparatus according to any foregoing claim further comprising means for comparing Doppler frequency shift spectra from serial examinations.

**Patentansprüche**

**1.** Vorrichtung zum Untersuchen eines Körpers lebenden Gewebes, das einen kanzerogen Tumor einhält, wobei die Vorrichtung eine optische Appliziereinrichtung, die eine Lichtquelle aufweist, um das Gewebe mit elektromagnetischer Strahlung zu bestrahlen, eine Strahlungsempfangeeinrichtung zum Empfangen der durch das Gewebe transmittierten Strahlung, eine Einrichtung zum Erzeugen eines Bildes der empfangenen Strahlung, eine Ultraschallanwendungseinrichtung (1, 2), um Ultraschall auf einen den Tumor umgebenden Bereich anzuwenden, eine Ultraschallempfangseinrichtung (3, 4), um reflektierten Ultraschall von einer sich ausbreitenden Vorderseite des kanzerogenen Tumors zu empfangen, eine Einrichtung zum Erzeugen eines Signals, das die Geschwindigkeit eines Blutflusses um den Tumor herum angibt, und eine Einrichtung, um aus dem Signal eine grafisch Wiedergabe zu erzeugen, die eine Angabe über eine Angiogenesis und einen zugeordneten Blutfluss bereitstellt, umfasst, **dadurch gekennzeichnet, dass** die Ultraschallanwendungseinrichtung (1, 2) in die optische Appliziereinrichtung integriert ist.

**2.** Vorrichtung nach Anspruch 1, bei der die Ultraschallanwendungseinrichtung (1, 2) und die Ultraschallempfangseinrichtung (3, 4) eine Ultraschall-Doppler-Blutfluss-Detektionseinrichtung umfassen, die piezoelektrische Sende- und Empfangselemente (1, 2, 3, 4) aufweist, die in ein Gehäuse eines Lichtleiters (5) der optischen Appliziereinrichtung aufgenommen sind.

**3.** Vorrichtung nach Anspruch 1 oder Anspruch 2, ferner mit einem Computer und einer Schaltvorrichtung, wobei die Schaltvorrichtung die Strahlungsempfangseinrichtung und die Ultraschallempfangseinrichtung (3, 4) mit dem Computer verbindet, wobei die Schaltvorrichtung wahlweise zwischen der Strahlungsempfangseinrichtung und der Ultraschallempfangseinrichtung (3, 4) umschaltbar ist, um dadurch auszuwählen, welche der Strahlungsempfangseinrichtung und der Ultraschallempfangseinrichtung (3, 4) mit dem Computer verbunden wird.

**4.** Vorrichtung nach einem des Anspruchs 1, Anspruchs 2 oder Anspruchs 3, bei der die Ultraschallanwendungseinrichtung (1, 2) und die Ultraschallempfangseinrichtung (3, 4) n piezoelektrische Elemente (1, 2, 3, 4) aufweisen, wobei n = 2, 4, 6, 8, 10, 12, ..., und die piezoelektrischen Elemente paarweise betrieben werden, um auf diese Weise Ultraschall-Doppler-Signale von einem hemisphärischen Gewebevolumen um den überwachten Tumor herum zu erhalten.

5. Vorrichtung nach Anspruch 4, bei der die piezoelektrischen Elemente (1, 2, 3, 4) in diagonal gegenüber liegenden Paaren betrieben werden.

6. Vorrichtung nach Anspruch 4, bei der die piezoelektrischen Elemente (1, 2, 3, 4) in benachbarten Paaren betrieben werden.

7. Vorrichtung nach einem der vorherigen Ansprüche, bei der die Ultraschallanwendungseinrichtung (1, 2) und die Ultraschallempfangseinrichtung (3, 4) piezoelektrische Elemente (1, 2, 3, 4) aufweisen und die Geometrie der piezoelektrischen Elemente (1, 2, 3, 4) in Form ringförmiger Elemente oder einer Reihe kreisförmiger Scheiben ist, von denen alle in einer Ebene in rechten winkeln zu einer optischen Achse der optischen Appliziereinrichtung liegen oder gegenüber dieser Ebene um Winkel von bis zu 45 Grad geneigt sein können.

8. Vorrichtung nach Anspruch 4 oder nach einem der Ansprüche 5 bis 7 soweit von Anspruch 4 abhängig, bei der die piezoelektrischen Elemente (1, 2, 3, 4) in abwechselnden Paaren betrieben werden und abwechselnde Paare bei 8 und 10 MHz oder 10 und 12 MHz oder anderen vergleichbaren Frequenzkombinationen betrieben werden.

9. Vorrichtung nach einem der vorherigen Ansprüche, ferner mit einer Inkrementierungseinrichtung zum schrittweisen Inkrementieren der elektromagnetischen optischen Strahlung von der Lichtquelle.

10. Vorrichtung nach einem der vorherigen Ansprüche, ferner mit einer Vergleichseinrichtung zum Vergleichen von Doppler-Frequenzverschiebungsspektren von Reihenuntersuchungen.

**Revendications**

1. Appareil pour examiner un corps constitué de tissu vivant contenant une tumeur cancéreuse, l'appareil comprenant un applicateur optique possédant une source lumineuse pour illuminer les tissus par des radiations électromagnétiques, un moyen de réception de radiations pour recevoir les radiations émises à travers les tissus, un moyen pour produire une image des radiations reçues, un moyen d'application d'ultrasons (1, 2) pour appliquer des ultrasons à une région entourant la tumeur, un moyen de réception d'ultrasons (3, 4) pour recevoir les ultrasons réfléchis par le front en progression de la tumeur cancéreuse, un moyen pour produire un signal qui soit représentatif de la vitesse du flux sanguin autour de la tumeur et un moyen pour générer, à partir du signal, une représentation graphique qui fournisse une indication de l'angiogénèse et du flux sanguin associé, **caractérisé en ce que** le moyen d'application d'ultrasons (1, 2) est incorporé dans l'applicateur optique.

2. Appareil selon la revendication 1, dans lequel le moyen d'application d'ultrasons (1, 2) et le moyen de réception d'ultrasons (3, 4) comprennent un détecteur de flux sanguin Doppler à ultrasons comprenant des éléments d'émission et de réception piézoélectriques (1, 2, 3, 4) qui sont incorporés dans le logement d'un guide de lumière (5) de l'applicateur optique.

3. Appareil selon la revendication 1 ou la revendication 2, comprenant en outre un ordinateur et un dispositif de commutation, le dispositif de commutation raccordant le moyen de réception de radiations et le moyen de réception d'ultrasons (3, 4) à l'ordinateur, le dispositif de commutation pouvant être commuté de manière sélective entre le moyen de réception de radiations (3, 4) et le moyen de réception d'ultrasons (3, 4), ce qui permet de sélectionner celui, du moyen de réception de radiations et du moyen de réception d'ultrasons, qui est raccordé à l'ordinateur.

4. Appareil selon l'une quelconque des revendication 1, revendication 2 et revendication 3, dans lequel le moyen d'application d'ultrasons (1, 2) et le moyen de réception d'ultrasons (3, 4) possèdent n éléments piézoélectriques (1, 2, 3, 4), où n = 2, 4, 6, 8, 10, 12, ..., lesdits éléments piézoélectriques étant actionnés par paires pour permettre l'obtention de signaux Doppler à ultrasons à partir d'un volume de tissu hémisphérique entourant la tumeur faisant l'objet d'une surveillance.

5. Appareil selon la revendication 4, dans lequel les éléments piézoélectriques (1, 2, 3, 4) sont actionnés par paires diagonalement opposées.

6. Appareil selon la revendication 4, dans lequel les éléments piézoélectriques (1, 2, 3, 4) sont actionnés par paires adjacentes.

7. Appareil selon l'une quelconque des revendications précédentes, dans lequel le moyen d'application d'ultrasons (1, 2) et le moyen de réception d'ultrasons (3, 4) comprennent des éléments piézoélectriques (1, 2, 3, 4), la géométrie des éléments piézoélectriques (1, 2, 3, 4) étant sous la forme de segments annulaires ou d'une série de disques circulaires, chacun d'eux pouvant être dans un plan à angle droit par rapport à l'axe optique de l'applicateur optique ou incliné par rapport à ce plan d'un angle ne dépassant pas 45 degrés.

8. Appareil selon la revendication 4, ou l'une quelconque des revendications 5 à 7 lorsque celles-ci dépendent de la revendication 4, dans lequel les éléments piézoélectriques (1, 2, 3, 4) sont actionnés par paires alternées, les paires alternées fonctionnant à 8 et 10 MHz, ou 10 et 12 MHz, ou selon d'autres combinaisons de fréquences similaires.

9. Appareil selon l'une quelconque des revendications précédentes, comprenant en outre un moyen d'incrémentation pour incrémenter par pas les radiations optiques électromagnétiques produites par la source lumineuse.

10. Appareil selon l'une quelconque des revendications précédentes, comprenant en outre un moyen pour comparer les spectres de décalage de fréquence Doppler à partir d'examens en série.

Figure 1

Figure 2

*Figure 3*

Figure 4

Figure 5